# EUROPEAN PATENT SPECIFICATION

(11) **EP 1 087 801 B1**
(45) Date of publication and mention of the grant of the patent: **16.01.2002**
(21) Application number: 99933575.5
(22) Date of filing: 24.06.1999
(51) Int. Cl.: A61L 29/00, A61L 31/00, A61K 31/47, A61K 31/57, A61K 31/165, A61K 31/275, A61K 31/335

(54) **TOPOISOMERASE INHIBITORS FOR PREVENTION OF RESTENOSIS**
TOPOISOMERASE INHIBITOREN ZUR RESTENOSE-PREVENTION
INHIBITEURS DE TOPOISOMERASE PERMETTANT DE PREVENIR LA RESTENOSE

(30) Priority: 26.06.1998 US 90764 P
(43) Date of publication of application: 04.04.2001
(73) Proprietor: Quanam Medical Corporation, Santa Clara, CA 95050 (US)
(72) Inventor: EURY, Robert, Cupertino, CA 95014 (US); ALVARADO, Angelica, Santa Clara, CA 95051 (US); POMERANTSEVA, Irina, Wakefield, MA 01880 (US); FROIX, Michael, Mountain View, CA 94040 (US)
(74) Representative: VOSSIUS & PARTNER
(86) International application number: US9914386
(87) International publication number: WO0000238

(56) References cited:
- WO-A-97/31003
- WO-A-97/33552
- WO-A-98/17331
- US-A- 5 383 928
- US-A- 5 569 197
- US-A- 5 674 192
- FUKUDA, M., NISHIO, K.: "Effects of combinations of CPT-II, paclitaxel and other anticancer agents on human small cell lung cancer cells" CELLULAR PHARMACOLOGY, vol. 3, no. 1, 1996, pages 1-6, XP002120621

## Description

### Field of the Invention

The present invention relates to a method of inhibiting cellular proliferation in a subject.

### Background of the Invention

Vascular disease is a leading cause of death and disability in westernized societies. Atherosclerosis is one of the more common forms of vascular disease and leads to insufficient blood supply to critical body organs resulting in heart attack, stroke and kidney failure. Atherosclerosis also causes complications in people suffering from hypertension and diabetes.

Atherosclerosis has been described as a form of vascular injury. A normal vessel wall consists of three reasonably well-defined layers: the intima, the media and the adventitia. The intima lines the lumen of all arteries and is composed of a single continuous layer of endothelial cells. The media consists of only one cell type, the smooth muscle cell, arranged in either a single layer or multiple lamellae. These cells are surrounded by small amounts of collagen and elastic fibers. The outermost layer of the artery is the adventitia, which consists of a loose interwoven admixture of collagen bundles, elastic fibers, smooth muscle cells and fibroblasts (Harrison's PRINCIPLES OF INTERNAL MEDICINE, 12th Edition, McGraw-Hill, Inc., 1991, Chapter 195).

While the processes causing atherosclerosis are complex and not completely understood, an underlying pathology to the numerous theories for the cause of atherosclerosis is the abnormal migration and proliferation of medial-smooth muscle cells into the intima (Harrison). The proliferation of the smooth muscle cells in the intima ultimately blocks blood flow and makes vessels abnormally susceptible to local blood clotting.

A similar pathology is also implicated in restenosis, the so-called recurrence of stenosis or artery stricture after corrective surgery. In fact, restenosis has been described as an accelerated atherosclerosis induced by injury (Forrester, J.S., *et al., JACC*, 17(3):758-769 (1991)). Restenosis results from vascular smooth muscle cell proliferation, migration and neo-intimal accumulation, due to incompletely understood processes involving regulatory molecules, such as platelet derived growth factor (Ferns, G.A, *et al., Science,* 253:1129 (1991)).

Restenosis has been observed to occur after coronary artery bypass surgery, heart transplantation, atherectomy, laser ablation and balloon angioplasty. In particular, restenosis is common after balloon angioplasty, also referred to as percutaneous transluminal coronary angioplasty, which is widely used as a treatment modality in patients with coronary artery disease to reduce lumen obstruction and improve coronary blood flow. It is estimated that between 25-35% of patients develop restenosis within 1-3 months after balloon coronary angioplasty, necessitating further interventions such as repeat angioplasty or coronary bypass surgery.

Therapies to reduce restenosis have focused on administration of chemotherapeutic agents which either interfere with formation of thrombosis or suppress smooth muscle cell proliferation. Anti-coagulants for suppression of thrombosis include heparin, warfarin, low molecular weight heparin and hirudin (Lovqvist, A., *et al*., *J. Int. Medicine,* 233:215-116 (1993)). Agents for inhibiting the proliferation of smooth muscle cells include glucocorticoids, angiotensin converting enzyme inhibitors, colchicine, vincristine, actinomycin, low molecular weight heparin, platelet derived growth factor and others (Lovqvist, A., *et al*.). More recently, paclitaxel (TAXOL® ) has been suggested for use in preventing restenosis (Kinsella, J.L. and Sollott, S. J., U.S. Patent No. 5,616,608, issued April 1, 1997).

However, of all the drugs tested, none have been found to be sufficiently effective, and some, such as colchicine, have been reported to be ineffective (O'Keefe, J.H., *et al., JACC*, 19(7):1597 (1992)).

### Summary of the Invention

Accordingly, it is an object of the present invention to provide a method of inhibiting cellular proliferation associated with restenosis and atherosclerosis.

In one aspect, the invention includes the use of a topoisomerase inhibitor for the preparation of a medicament for inhibiting cell proliferation associated with a hyper proliferative condition.

In one embodiment, the topoisomerase inhibitor is selected from the group consisting of camptothecin, irinotecan and topotecan.

In one embodiment, the hyperproliferative condition is restenosis.

In one embodiment, administration can be effected by local administration of the topoisomerase inhibitor. Local administration of the drug is, in one embodiment, by means of a drug delivery catheter, or in another embodiment, by a guidewire.

In another embodiment, the topoisomerase inhibitor is locally administerable to the treatment site via a stent carrying the topoisomerase inhibitor. The stent, in one embodiment, is a polymer stent loaded with a topoisomerase inhibitor selected from the group consisting of camptothecin, irinotecan and topotecan.

In other embodiments, the stent is a metal stent and the topoisomerase inhibitor is incorporated into a polymer sheath carried on the metal stent, or the metal stent is coated with a synthetic polymer or a biopolymer carrying the topoisomerase inhibitor, or the metal stent includes surface indentations in which the topoisomerase inhibitor is incorporated.

In another embodiment, the method includes coadministering a second therapeutic agent with the topoisomerase inhibitor. The second therapeutic agent, in one embodiment, is verapamil, dexamethasone or a microtubule stabilizing agent such as paclitaxel, derivatives of paclitaxel, and colchicine. In another embodiment, the second therapeutic agent is radiation therapy.

In another aspect, the invention includes the use of an effective amount of a topoisomerase inhibitor for preparing a mearcamert for inhibiting restenosis in a patient.

In another aspect, the invention includes a device for treatment of restenosis, comprising a stent carrying a therapeutically effective amount of a topoisomerase inhibitor.

In one embodiment, the stent is a polymer stent loaded with a topoisomerase inhibitor selected from camptothecin, irinotecan and topotecan.

In another embodiment, the stent is a metal stent and the topoisomerase inhibitor is incorporated into a polymer sheath carried on the metal stent. In another embodiment, the metal stent is coated with a synthetic polymer or a biopolymer carrying the topoisomerase inhibitor. In a further embodiment, the metal stent has surface indentations and the topoisomerase inhibitor is incorporated into the pits.

The stent, in another embodiment, includes a second therapeutic agent for treatment of restenosis. The second therapeutic agent, in one embodiment, is a microtubule stabilizing agent such as paclitaxel, derivatives of paclitaxel, and colchicine or the agent is verapamil or dexamethasone.

These and other objects and features of the invention will be more fully appreciated when the following detailed description of the invention is read in conjunction with the accompanying drawings.

### Brief Description of the Drawings

Figs. 1A-1C are illustrations of the basic V-shaped stent used in studies in support of the present invention, where the stent is shown unwound (Fig. 1A), wound to a small diameter for insertion in a vessel (Fig. 1B) and in an open, expanded diameter after placement in a vessel (Fig. 1C); and
Figs. 2A-2B are artist-renderings of angiogram images of pig coronary arteries one month after insertion of a camptothecin-loaded stent in accordance with the invention (Fig. 2A) or of a metal, control stent (Fig. 2B).

### Detailed Description of the Invention

### I. Definitions

"Hyperproliferative condition" refers to undesirable cell growth associated with atherosclerosis, restenosis, proliferative vitreoretinopathy and psoriasis. The term is not intended to include cellular hyperproliferation associated with cancerous conditions.

"Undesirable cell growth" or "inhibiting undesirable cell growth" refers to unregulated cell division associated with smooth muscle cells or fibroblasts and to the inhibition of such growth.

"Topoisomerase inhibitor" refers to any compound that inhibits the action of topoisomerase enzymes, including topoisomerase I and topoisomerase II. Such inhibitors include camptothecin, derivatives and analogs of camptothecin, such as irinotecan, topotecan and 9-amino-camptothecin, etoposide, teniposide, genistein and mitoxantron.

"Administering" as referred to herein is intended to include routes of administration which allow the topoisomerase inhibitor to perform its intended function of inhibiting undesirable cell growth. Such administering includes systemic and local or site specific administration by an appropriate route, such as injection (subcutaneous, intravenous, parenteral, intraperitoneal, intrathecal, etc.) oral, inhalation, transdermal, administration by means of a drug delivery catheter, or implantation of a drug-carrying device.

"Effective amount" refers to the amount necessary or sufficient to inhibit the undesirable cell growth, *e.g.*, prevent the undesirable cell growth or reduce the existing cell growth. The effective amount can vary depending on factors known to those of skill in the art, such as the type of cell growth, the mode and regimen of administration, the size of the subject, the severity of the cell growth, etc. One of skill in the art would be able to consider such factors and make the determination regarding the effective amount.

"Pharmaceutically acceptable carrier" refers to any substance coadministered with the topoisomerase inhibitor which allows the compound to perform its intended function. Examples of such carriers include solutions, solvents, dispersion media, delay agents, emulsions, microparticles and the like.

### II. Use

In the use of the invention, a topoisomerase inhibitor is administerable to a subject at risk of developing or suffering from a hyperproliferative condition. The topoisomerase inhibitor is administerable in a therapeutically effective amount by a selected route as will be described.

Preferred topoisomerase inhibitors for use according to the invention include camptothecin and analogs thereof. Camptothecin is a pentacyclic alkaloid initially isolated from the wood and bark of *Camptotheca acuminata*, a tree indigenous to China (Wall, M.E. *et al*., *J. Am. Chem. Soc.,* 94:388 (1966)). Camptothecin exerts its pharmacological effects by irreversibly inhibiting topoisomerase I, an enzyme intimately involved in DNA replication. Methods for the synthesis of camptothecin and camptothecin analogs are known, and are summarized and set forth in U.S. Patent No. 5,244,903, which is herein incorporated by reference in its entirety.

Analogues of camptothecin include SN-38 ((+)-(4S)-4,11-diethyl-4,9-dihydroxy-1H-pyrano[3',4' :6,7]-indolizino [1,2-b]quinoline-3,14(4H,12H)-dione); 9-aminocamptothecin; topotecan (hycamtin; 9-dimethyl-aminomethyl-10-hydroxycamptothecin); irinotecan (CPT-11; 7-ethyl-10-[4-(1-piperidino)-1-piperidino]-carbonyloxy-camptothecin), which is hydrolyzed *in vivo* to SN-38); 7-ethylcamptothecin and its derivatives (Sawada, S. *et al*., *Chem. Pharm*. *Bull.,* 41(2):310-313 (1993)); 7-chloromethyl-10,11-methylene-dioxy-camptothecin; and others (SN-22, Kunimoto, T. *et al*., *J. Pharmacobiodyn*., 10(3):148-151: (1987); DX-8951f and GG-211 Rothenberg, M.L., *Ann*. *Oncol.,* 8(9):837-855 (1997)).

Other topoisomerase inhibitors for use according to the invention include 4'-(acridinylamino)-methanesulfon-m-anisidide (amsacrine) and its analogues, such as N-5-dimethyl-9-(2-methoxy-4-methylsulfonylamino)-phenylamino-4-acridinecarboxamide (CI-921, Traganos, F., *et al*., *Cancer Res.,* 47(2):424-432 (1987)) and methyl-N-[4-9-acridinylamino)-2-methoxyphenyl]carbamate hydrochloride (m-AMCA, Moreland, N., *et al*., *Eur*. *J*. *Cancer,* 33(10):1668-1676 (1997)); 6-N-formylamino-12,13,dihydro-1,11-dihydroxy-13-(beta-D-glucopyranosyl)-5H-indolo[2,3-a]pyrrolo[3,4-c]carbazole-5,7(6H)-dione (NB-506, Kanzawa, F *et al*., *Cancer Res.,* 55(13):2806-2813 (1995); etoposide (VP-16, Karato, A., *et al*., *J. Clin*. *Oncol.,* 11(10):2030-2035 (1993)); m-AMSA (Nakano, H., *Gan To Kagaku Ryoho,* 18(10):1550-1555 (1991)); 6-[[2-(dimethylamino)-ethyl]amino]-3-hydroxy-7H-indeno[2,1-c]quinolin-7-one dihydrochloride, (TAS-103, Utsugi, T., *et al., Jpn. J. Cancer Res.,* 88(10):992-1002 (1997)); azatoxin (Solary E., *et al*., *Biochem. Pharmacol*., 45(12):2249-2456 (1993)); 3-methoxy-11H-pyrido[3',4'-4,5]pyrrolo[3,2-c]quinoline-1,4-dione (AzalQD, Riou, J.F., *et al*., *Mol. Pharmacol*., 40(5):699-706 (1991)); anthracenyl-amino acid conjugates (ICRF 506, Meikle, I., *et al*., *Biochem. Pharmacol*., 49(12):1747-1757 (1995)); and bis(2,6-dioxopiperazine derivatives (ICRF-154, ICRF-193, ICRF-159 and MST-16, Kizaki, H., *et al*., *Adv. Enzyme Regul.,* 37:403-423 (1997)).

In one embodiment of the invention, the topoisomerase inhibitor administered is the pharmacologically active enantiomer of a camptothecin analogue having a chiral center. The enantiomer can be resolved from the racemic mixture using techniques known to those of skill in the art.

In another embodiment, the topoisomerase inhibitor is camptothecin or an analogue of camptothecin and is administered in combination with a second therapeutic compound selected from paclitaxel, and derivatives of paclitaxel, calcium channel blockers, such as verapamil, and steroidal non-inflammatory agents, such as dexamethasone. When the second therapeutic agent is a calcium channel blocker, it serves to reduce elimination of the first therapeutic agent from the cells. When the second therapeutic agent is an antiinflammatory agent, it is effective to reduce inflammation at a site of injury.

The therapeutic compounds described above are used for inhibiting the growth of vascular smooth muscle cells in the vessel. "Inhibiting" as used herein is intended to include reducing, delaying or eliminating undesirable cell growth. With respect to the embodiment of the invention where the undesirable cell growth is associated with restenosis following balloon coronary angioplasty, "reducing" means decreasing the intimal thickening that results from smooth muscle cell proliferation following angioplasty, either in an animal model or in humans. "Delaying" means delaying the time until onset of visible intimal hyperplasia, as observed histologically or by angiographic techniques, following angioplasty. "Eliminating" refers to completely reducing and/or completely delaying intimal hyperplasia in a subject such that sufficient blood flow in the vessel is established and surgical intervention is not necessary.

The topoisomerase inhibitors are administered in accordance with the invention by any route which provides effective therapy for the inhibition of restenosis. Such routes include but are not limited to systemic administration of the drug by injection, including bolus, pulsed and continuous administration injected intravenously, subcutaneously, intramuscularly, intraperitoneally, etc. Continuous or delayed release formulations are also contemplated, both for systemic administration and local or site specific administration.

One preferred mode of administering the topoisomerase inhibitor is via a drug delivery catheter, such as those described in U.S. Patent Nos. 5,558,642, 5,295,962, 5,171,217 and 5,674,192. Typically, such catheters have a flexible shaft and an inflatable balloon at the distal end of the shaft. The catheter is inserted into a vessel in an un-inflated condition and the balloon is positioned at the site to be treated with the topoisomerase inhibitor. The balloon member is inflated and apertures in the balloon assembly provide drug carried in the catheter to be delivered to the target site. The drug can be carried in solution form, entrapped in microparticles of a physiologically compatible polymer or incorporated into a polymer, such as a hydrogel, which is coated on the balloon region for rapid release of the drug during expansion of the balloon. Catheters such as these provide a convenient way to administer the drug in conjunction with a balloon angioplasty procedure.

In other embodiments, the topoisomerase inhibitor is administered to the target site by an infusion catheter or by a drug delivery guidewire. An infusion catheter provides delivery of agents to a target site by placing the tip of the catheter at the site and connecting the catheter to a pump. The tip of the catheter generally includes openings through which the agent is pumped at a desired rate to the target site (U.S. Patent No. 5,720,720). A drug delivery guidewire has been described in U.S. Patent No. 5,569,197, where the guidewire is hollow and has an opening at its distal end for infusion of a drug therethrough.

In a preferred embodiment, the topoisomerase inhibitor is administered locally to the site of undesired cell growth in the form of an implanted medical device, such as a stent. Endovascular stents for use following balloon angioplasty are known in the art and described in, for example, U.S. Patent Nos. 5,395,390 (Simon), 4,739,762 (Palmaz), 5,195,984 (Schatz) and 5,163,952 (Froix).

In one embodiment, the stent is a metal stent. Exemplary biocompatible and nontoxic metals for stents include nickel-titanium alloys, tantalum, and steel. In this embodiment, the topoisomerase inhibitor can be adsorbed onto the stent or incorporated into indentations, *i.e.,* pockets, grooves or pits, formed on the surface of the stent. In another embodiment, the metal stent is coated with a polymer-drug solution containing the selected topoisomerase inhibitor or drug combination by dipping the stent in the solution or spraying the stent with the solution.

The metal stent, in other embodiments, is adapted to carry a polymer member, where the metal stent serves as a structural support for the polymer member carrying the topoisomerase inhibitor. For example, a polymer-based, drug-containing fiber can be threaded through the stent apertures. The metal stent provides the mechanical support in the vessel after deployment for maintaining vessel patency, and the polymer thread provides a controlled release of the topoisomerase inhibitor. Another example is to provide a drug-loaded polymer sheath for encompassing the stent, as described in U.S. Patent No. 5,383,928 (Scott, *et al*.). Another example is to provide a polymer stent which coexpands with the metal stent when placed in the target vessel, as described in U.S. Patent No. 5,674,242 (Phan, *et al*.).

In another embodiment, the stent is formed of a biocompatible polymer, including hydrogels, polyurethanes, polyethylenes, ethylenevinyl acetate copolymers, and the like. One preferred class of polymers are shape-memory polymers, as described for example by Froix, U.S. Patent No. 5,163,952, which is incorporated by reference herein. Stents formed of shape-memory polymers, which include methacrylate-containing and acrylate-containing polymers, readily expand to assume a memory condition to expand and press against the lumen walls of a target vessel, as described by Phan, U.S. Patent No. 5,603,722, which is incorporated by reference in its entirety.

In studies in support of the present invention, the topoisomerase inhibitor camptothecin was incorporated into polymer stents and implanted into pig coronary arteries. In other studies, polymer stents containing etoposide, amsacrine, mycophenolic acid, dipyridamole and dexamethasone were prepared and implanted into pig coronary arteries. These studies will now be discussed.

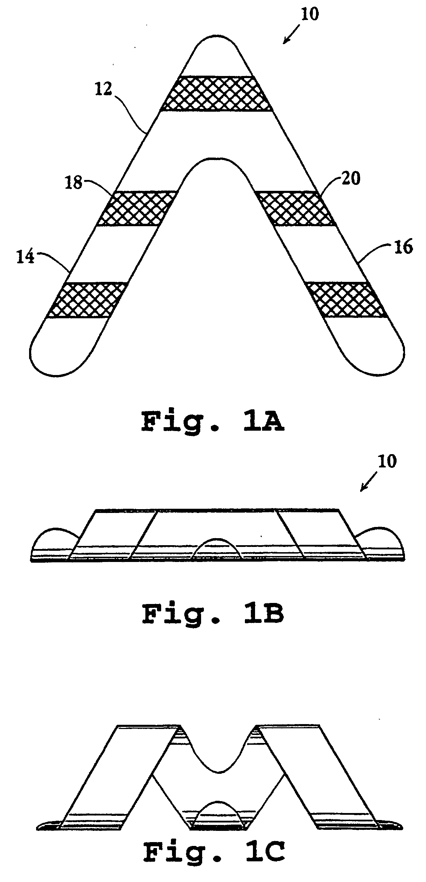

The polymer stent used in the studies in support of the invention is illustrated in Figs. 1A-1C. Stent 10 is composed of a unitary strip 12 having two legs 14, 16. The stent typically includes a radio-opaque material, such as gold, stainless steel, platinum, tantalum or metal salts, to provide a means of identifying by x-ray or other imaging technique the location of the stent during and after stent placement. Stent 10 in Fig. 1A includes bands of gold 18, 20 for imaging purposes. The radio-opaque material is incorporated into the stent prior to formation of the polymer or is applied as a coating after formation of the stent.

Fig. 1B shows stent 10 placed in a closed condition for insertion and placement in a target vessel. To place the stent in its small diameter, closed condition, the stent is wound around a cylinder or rod sized according to the diameter of the target vessel. For example, the stent of Fig. 1A can be wrapped around the balloon portion of a balloon catheter and secured thereon by a variety of means, including restraining devices, adhesives or, in the case of memory polymers, by the self-restraining nature of the material. Stent 10 is wound into its closed condition by wrapping legs 14, 16 in the same direction or in opposite directions.

After placement of the stent in a target vessel, the stent is expanded by a stimulus, such as pressure from the balloon portion of the catheter or heat. The leg portions of the stent expand radially until their movement is constrained by the walls of the vessel.

Stents as illustrated in Fig. 1 were prepared as described in Example 1 and were composed primarily of methylmethacrylate and polyethyleneglycol methacrylate. The stents were loaded with camptothecin by applying a concentrated solution of the drug to the surface of the stent.

Camptothecin-containing stents were inserted into the coronary arteries of pigs, as set forth in Examples 2-4. In the study described in Example 2 and 3, a polymer stent was loaded with 1 µg camptothecin from a solution of the drug in dimethylformamide. The polymer stent was deployed into the pig artery using a balloon catheter. As controls, a commercially available metal stent was inserted and a metal stent carrying a v-shaped polymer stent of the same composition as the test stent, except that it was free of camptothecin.

At the time of insertion of the test and control stents, the coronary artery was characterized using a computer-based coronary angiography analysis system (Umans, V.A., *et al*., *JACC,* 21(6):1382-1390 (1993)). Boundaries of a selected coronary artery segment were detected automatically from optically magnified and video-digitized regions of interest. The catheter used for insertion of the stents was used as a scaling device to determine the dimensions of the artery at the site of implantation. The original vessel diameter at the time of implantation was determined.

The test stent and the control stents were left in place in the pig artery for one month. The arteries were then explanted from the pig and pressure fixed for morphometric analysis. The minimal lumen diameter of the vessel after the one month treatment period was found by determining the smallest lumen diameter in the region of stent placement. "Late loss" was calculated by subtracting the minimal lumen diameter post treatment from the minimum inner stent diameter. The percent stenosis was taken as the late loss divided by the original vessel diameter times 100.

The camptothecin eluting polymer stent had a late loss of 0.3 mm and a percent diameter stenosis of 10%. The metal stent control had a late loss of 0.49 mm and a percent diameter stenosis of 16%. The metal stent carrying the drug-free polymer stent had a late loss of 1.46 mm and a percent diameter stenosis of 45%. These results are summarized in Tables 2 and 3 in Examples 2 and 3, respectively.

In another study, described in Example 4, a camptothecin-eluting polymer stent was prepared by loading the stent with 56 µg drug from an N-methylpyrrolidone solution. The stent and a metal control stent were inserted into pig coronary arteries, as described above and in Example 4, for a one month period. The coronary arteries were explanted and angiographic images of the arteries with the stents in place were taken.

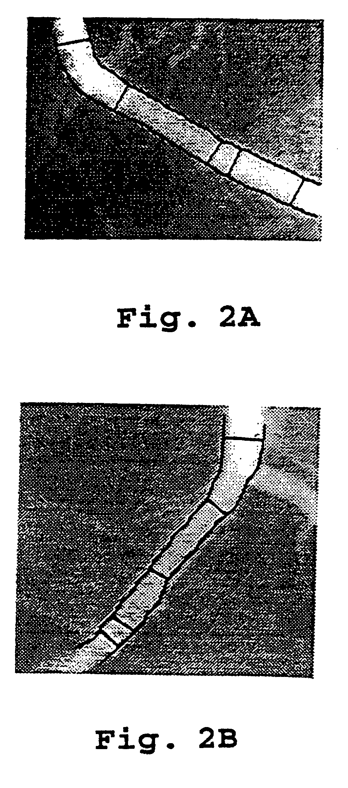

Fig. 2A is a rendering of the angiographic image of the artery treated with the camptothecin-eluting polymer stent of the invention. Fig. 2B shows a rendering of the angiographic image of the artery treated with the metal stent. The narrowing as a result of stenosis in the artery treated with the metal stent (Fig. 2B) is apparent by comparing the figures. Late loss and percent diameter stenosis were determined for the stents and are summarized in Table 4 in Example 4. The metal stent had a late loss of 1.0 mm and a percent diameter stenosis of 38%. The camptothecin-eluting stent had a late loss of 0.5 mm and a percent diameter stenosis of 16%.

These studies clearly demonstrate that camptothecin is effective to inhibit the undesirable cell growth associated with vessel injury during coronary angioplasty and stent insertion.

In other studies in support of the invention, polymer stents were prepared and loaded with etoposide (Example 5), amsacrine (Example 6) and mycophenolic acid (Example 7). Each of these compounds were successfully loaded into polymer stents.

The loading level of drug into the stent can be selected and tailored according to the desired treatment regimen. The loading is readily varied, as will be appreciated by one of skill in the art, by varying the loading solvent, the drug concentration and the polymer composition of the stent. Typically loading levels are between 0.01-50% drug on a weight basis. For camptothecin, the loading level is readily varied between 0.1-10%, with the preferred loading level for effective therapy between about 0.2-9%.

In another embodiment of the invention, a second therapeutic agent is administered along with the topoisomerase inhibitor. The second therapeutic agent, in one embodiment is radiation, and in another embodiment, is a therapeutic agent which is incorporated into the stent or is administered by another route, such as a systemic or local route of administration described above. Exemplary compounds for use as the second therapeutic agent include paclitaxel, derivatives of paclitaxel including water soluble and non-water soluble derivatives, verapamil, colchicine and dexamethasone.

### III. EXAMPLES

The following examples illustrate a method of administering a topoisomerase inhibitor, camptothecin, to a subject, in accordance with the present invention. The examples are in no way intended to limit the scope of the invention.

### A. Materials

Camptothecin, etoposide (4'-desmethylepipodophyllotoxin 9-[4,6-o-ethylidene-β-Dglucopyranoside), amsacrine (4-[9-acridinylamino]-N-[methane-sulfonyl]-m-anisidine), mycophenolic acid (6-[4-hydroxy-6-methoxy-7-methyl-3-oxo-5-phthalanyl]-4-methyl-4-hexanoic acid), dipyridamole and dexamethasone were purchased from Sigma (St. Louis, MO). All solvents were reagent grade.

### B. Methods

Late loss was calculated by subtracting the inner diameter of the stent from the measured minimal lumen diameter of the vessel after the one-month treatment period with a stent. Percentage stenosis was taken as the late loss divided by the original vessel diameter x 100.

### EXAMPLE 1

### Polymer Stent Preparation

A polymer stent is prepared according to the procedure described in U.S. Patent No. 5,674,242 (Phan, *et al*.). Briefly, the materials in Table 1 were mixed together in the specified amounts, purged with nitrogen, and then polymerized between glass plates to form thin films having a thickness of approximately 0.14 mm. Prior to polymerization, gold strips were placed at intervals to provide for radio-opacity of the stents.

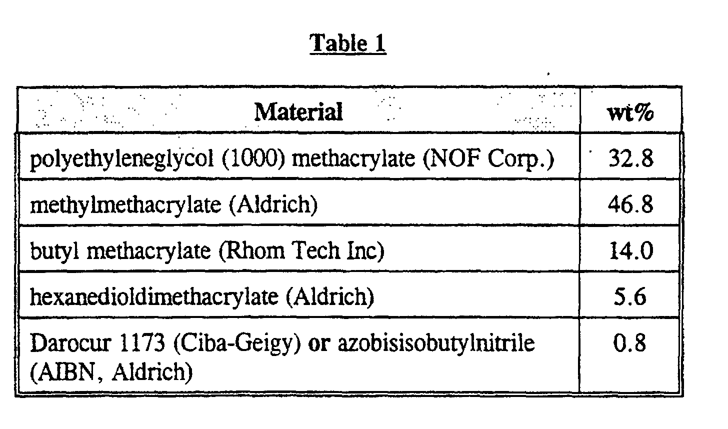

After polymerization, the film was cut into V-shaped strips (Fig. 1) using a punch and any unpolymerized monomer was removed by solvent extraction.

The selected drug was loaded into the polymer stent by preparing a solution of the drug in a suitable solvent, typically an alcohol (isopropanol or methanol), n-methylpyrrolidone or dimethylformamide. The stent was weighed and placed in a clean container. A known volume of the drug solution was pipetted over the surface of the stent. The stent was then placed in a vacuum oven at about 40 °C for between 1-3 days to dry.

### EXAMPLE 2

### Camptothecin-Containing Polymer Stent

A V-shaped polymer stent was prepared as described in Example 1. The stent was loaded with camptothecin as follows. 5.4 mg camptothecin was dissolved in 27 ml dimethylformamide and 5 µl of this drug solution was pipetted over the surface of the stent to obtain a loading of 1 µg in the stent.

The camptothecin-containing polymer stent and, as a control, a commercially available metal, corrugated-ring type stent were placed into the coronary arteries of a healthy Domestic Farm Swine pig (Pork Power, Inc.) by conventional techniques using a commercially available catheter (Advanced Cardiovascular Systems). At the time of insertion, the coronary artery was characterized using a computer-based coronary angiography analysis system (Umans, V.A., *et al*., *JACC*, 21(6):1382-1390 (1993)). Boundaries of the coronary artery segment were detected automatically from optically magnified and video-digitized regions of interest. The catheter used for insertion of the stents was used as a scaling device to determine the dimensions of the artery at the site of implantation. The original vessel diameter at the time of implantation was determined. The stents were imaged during and after the insertion procedure to ensure proper placement.

One month after placement of the test stent and the control stent, the pig was euthanized and the heart and coronary arteries explanted. The arteries were pressure fixed for morphometric analysis. The minimal lumen diameter of the vessel after the one month treatment period was found by determining the smallest lumen diameter in the region of stent placement. "Late loss" was calculated by subtracting the minimal lumen diameter post treatment from the minimum inner stent diameter. The percent stenosis was taken as the late loss divided by the original vessel diameter times 100. The late loss and percent stenosis are shown in Table 2.

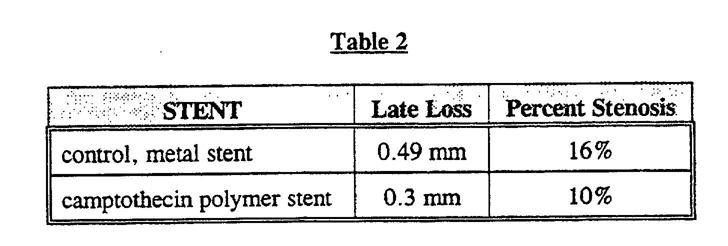

### EXAMPLE 3

### Camptothecin-Containing Polymer Stent

A camptothecin-containing polymer stent was prepared as described in Example 2. The control metal stent was wrapped with a similar, but camptothecin-free V-shaped polymer stent. Both stents were inserted into the coronary arteries of a pig, as described in Example 2 and explanted after one month. The late loss and percent restenosis are shown in Table 3.

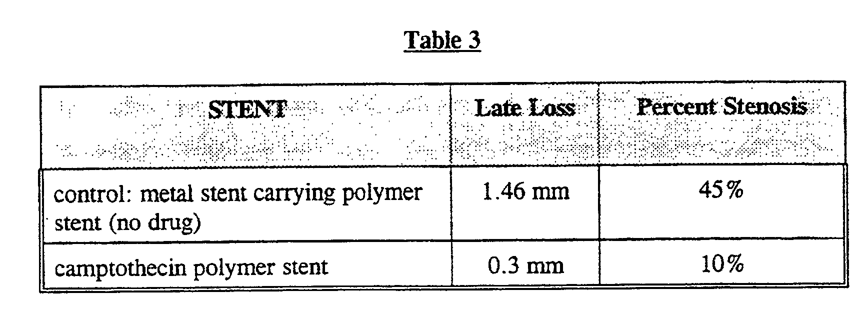

### EXAMPLE 4

### Camptothecin-Containing Polymer Stent

A V-shaped polymer stent was prepared as described in Example 1 and was loaded with camptothecin by the following procedure. 50.2 mg camptothecin was dissolved in 4.5 ml N-methylpyrrolidone and 5 µl of this solution was pipetted over the surface of the stent to obtain a loading of 56 µg. The stent was dried in a vacuum oven at 40 °C for 3 days.

The camptothecin-containing polymer stent and, as a control, a commercially available metal, corrugated ring-type stent were placed into the coronary arteries of a healthy Domestic Farm Swine pig (Pork Power, Inc.) by conventional techniques using a commercially available catheter (Advanced Cardiovascular Systems). The stents were imaged during and after the insertion procedure to ensure proper placement using convention angiographic imaging techniques.

One month after placement of the test stent and the control stent, the pig was euthanized and the heart and coronary arteries explanted. The arteries were pressure fixed for morphometric analysis. Images of the coronary arteries containing the polymer camptothecin stent and the control metal stent are shown in Figs. 2A and 2B, respectively. The late loss and percent stenosis are shown in Table 4.

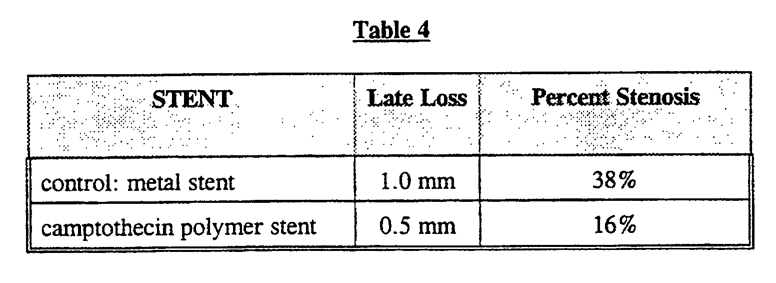

### EXAMPLE 5

### Etoposide-Containing Polymer Stent

A V-shaped polymer stent was prepared as described in Example 1 and loaded with etoposide as follows. 0.0251 grams of etoposide was weighed into a polypropylene test tube with cap. 500 µl of dimethylformamide was added and the tube agitated until the etoposide was dissolved.

The stent was loaded with etoposide by the method described above in the methods section, by placing 5 µl over the V-shaped stent and drying the stent in an oven for 24 hours. The loading was 250 µg/stent.

### EXAMPLE 6

### Amsacrine-Containing Polymer Stent

Five V-shaped polymer stents were prepared as described in Example 1. A solution containing 10 mg of amsacrine in 300 µl of dimethylformamide was prepared and mixed until the amsacrine was dissolved.

The five stents were loaded with amsacrine by placing 5 µl of the drug solution over the surface of each V-shaped stent and drying the stents in an oven for 24 hours. The loading procedure was repeated two times more to achieve a drug loading of 500 µg/stent.

### EXAMPLE 7

### Mycophenolic Acid-Containing Polymer Stent

Four V-shaped polymer stents were prepared as described in Example 1. A solution containing 53.35 mg of mycophenolic acid in 500 µl of dimethylformamide was prepared and agitated until the mycophenolic acid was dissolved.

The four stents were loaded with mycophenolic acid by placing 5 µl of the drug solution over the surface of each V-shaped stent and drying the stents in an oven for 24 hours. The drug loading was about 534 µg/stent.

### EXAMPLE 8

### Polymer Stent Containing Camptothecin and Dexamethasone

A V-shaped polymer stent is prepared as described in Example 1. The stent is loaded with camptothecin and dexamethasone by pipetting 5 µl of a solution containing camptothecin and dexamethasone in dimethylformamide over the stent. The stent is dried in an oven for 24 hours.

Although the invention has been described with respect to particular embodiments, it will be apparent to those skilled in the art that various changes and modifications can be made without departing from the invention.

## Claims

1. Use of a topoisomerase inhibitor for the preparation of a pharmaceutical composition for inhibiting cellular proliferation associated with a hyperproliferative condition in a subject whereby cellular hyperproliferation associated with cancerous conditions is excluded.

2. The use according to claim 1, wherein the hyperproliferative condition is restenosis.

3. The use according to claim 1 or claim 2, wherein the topoisomerase inhibitor is locally delivered via a drug delivery catheter, a guidewire or a stent.

4. The use according to any one of claims 1-3, wherein the topoisomerase inhibitor is selected from the group consisting of camptothecin, irinotecan and topotecan.

5. The use according to claim 4, wherein the topoisomerase inhibitor is incorporated into a polymer sheath carried on a metal stent.

6. The use according to claim 5, wherein the stent is coated with a synthetic polymer or a biopolymer carrying the topoisomerase inhibitor.

7. The, use according to claim 4, wherein the topoisomerase inhibitor is incorporated into indentations formed on a metal stent.

8. The use according to any of claims 1-7 wherein the pharmaceutical composition further includes a second therapeutic agent.

9. The use according to claim 8, wherein said second therapeutic agent is a microtubule stabilizing agent.

10. The use according to claim 9, wherein said microtubule stabilizing agent is selected from paclitaxel, derivatives of paclitaxel, verapamil, colchicine and dexamethasone.

11. The use according to claims 1-7, wherein said composition is used together with radiation treatment.

12. A device for treatment of restenosis, comprising a stent carrying a therapeutically effective amount of a topoisomerase inhibitor.

13. The device of claim 12, wherein said stent is a polymer stent loaded with a topoisomerase inhibitor selected from the group consisting of camptothecin, irinotecan and topotecan.

14. The device of claim 12, wherein the stent is a metal stent and the topoisomerase inhibitor is incorporated into a polymer sheath carried on the metal stent.

15. The device of claim 12, wherein the stent is coated with a synthetic polymer or a biopolymer carrying the topoisomerase inhibitor.

16. The device of claim 12, wherein the stent is a metal stent and the topoisomerase inhibitor is incorporated into indentations formed in the stent.

17. The device of claim 12, which further includes a second therapeutic agent for treatment of restenosis.

18. The device of claim 17, wherein said second therapeutic agent is a microtubule stabilizing agent.

19. The device of claim 18, wherein said microtubule stabilizing agent is selected from the group consisting of paclitaxel, derivatives of paclitaxel, verapamil colchicine and dexamethasone.

## Patentansprüche

1. Verwendung eines Topoisomerase-Inhibitors für die Herstellung eines Arzneimittels zur Hemmung der zellulären mit einem hyperproliferativen Zustand in Zusammenhang stehenden Proliferation in einem Patienten, wobei zelluläre Hyperproliferation, die mit einem Krebszustand in Zusammenhang steht, ausgeschlossen ist.

2. Verwendung nach Anspruch 1, wobei der hyperproliferative Zustand Restenose ist.

3. Verwendung nach Anspruch 1 oder 2, wobei der Topoisomerase-Inhibitor über einen Arzneistoffverteilungskatheter, einen Führungsdraht oder einen Stent örtlich verteilt wird.

4. Verwendung nach einem der Ansprüche 1 bis 3, wobei der Topoisomerase-Inhibitor ausgewählt ist aus der Gruppe bestehend aus Camptothecin, Irinotecan und Topotecan.

5. Verwendung nach Anspruch 4, wobei der Topoisomerase-Inhibitor in eine Polymerschicht auf einem Metallstent eingebaut ist.

6. Verwendung nach Anspruch 5, wobei der Stent mit einem den Topoisomerase-Inhibitor tragenden synthetischen Polymer oder Biopolymer beschichtet ist.

7. Verwendung nach Anspruch 4, wobei der Topoisomerase-Inhibitor in auf einem Metallstent ausgebildeten Vertiefungen eingebaut ist.

8. Verwendung nach einem der Ansprüche 1 bis 7, wobei das Arzneimittel weiterhin einen zweiten therapeutischen Wirkstoff umfasst.

9. Verwendung nach Anspruch 8, wobei der zweite therapeutische Wirkstoff ein Mikrotubulus-stabilisierender Wirkstoff ist.

10. Verwendung nach Anspruch 9, wobei der Mikrotubulus-stabilisierende Wirkstoff ausgewählt ist aus Paclitaxel, Derivaten von Paclitaxel, Verapamil, Colchicin und Dexamethason.

11. Verwendung nach einem der Ansprüche 1 bis 7, wobei das Arzneimittel zusammen mit einer Bestrahlungsbehandlung verwendet wird.

12. Vorrichtung zur Behandlung von Restenose, umfassend einen Stent, der eine therapeutisch wirksame Menge eines Topoisomerase-Inhibitors trägt.

13. Vorrichtung nach Anspruch 12, wobei der Stent ein Polymerstent ist, der mit einem Topoisomerase-Inhibitor ausgewählt aus der Gruppe bestehend aus Camptothecin, Irinotecan und Topotecan beladen ist.

14. Vorrichtung nach Anspruch 12, wobei der Stent ein Metallstent ist und der Topoisomerase-Inhibitor in eine Polymerschicht auf dem Metallstent eingebracht ist.

15. Vorrichtung nach Anspruch 12, wobei der Stent mit einem den Topoisomerase-Inhibitor tragenden synthetischen Polymer oder Biopolymer beschichtet ist.

16. Vorrichtung nach Anspruch 12, wobei der Stent ein Metallstent ist und der Topoisomerase-Inhibitor in auf dem Stent ausgebildeten Vertiefungen eingebracht ist.

17. Vorrichtung nach Anspruch 12, das weiterhin einen zweiten therapeutischen Wirkstoff zur Behandlung von Restenose einschließt.

18. Vorrichtung nach Anspruch 17, wobei der zweite therapeutische Wirkstoff ein Mikrotubulus-stabilisierender Wirkstoff ist.

19. Verwendung nach Anspruch 18, wobei der Mikrotubulus-stabilisierende Wirkstoff ausgewählt ist aus der Gruppe bestehend aus Paclitaxel, Derivaten von Paclitaxel, Verapamil, Colchicin und Dexamethason.

## Revendications

1. Utilisation d'un inhibiteur de topoisomérase pour la préparation d'une composition pharmaceutique pour inhiber une prolifération cellulaire associée à un état hyperprolifératif chez un sujet de telle manière que la prolifération cellulaire associée à des états cancéreux soit éliminée.

2. Utilisation selon la revendication 1, dans laquelle l'état hyperprolifératif est la resténose.

3. Utilisation selon la revendication 1 ou la revendication 2, dans laquelle l'inhibiteur de topoisomérase est administré localement par l'intermédiaire d'un cathéter d'administration de médicaments, un fil guide ou un extenseur.

4. Utilisation selon l'une quelconque des revendications 1 à 3, dans laquelle l'inhibiteur de topoisomérase est choisi dans le groupe constitué par la camptothécine, l'irinotécane et le topotécane.

5. Utilisation selon la revendication 4, dans laquelle l'inhibiteur de topoisomérase est incorporé dans une gaine de polymère supportée par un extenseur métallique.

6. Utilisation selon la revendication 5, dans laquelle l'extenseur est revêtu d'un polymère synthétique ou d'un biopolymère portant l'inhibiteur de topoisomérase.

7. Utilisation selon la revendication 4, dans laquelle l'inhibiteur de topoisomérase est incorporé dans des entailles formées sur un extenseur métallique.

8. Utilisation selon l'une quelconque des revendications 1 à 7, dans laquelle la composition pharmaceutique comprend, en outre, un deuxième agent thérapeutique.

9. Utilisation selon la revendication 8, dans laquelle ledit deuxième agent thérapeutique est un agent stabilisant microtubulaire.

10. Utilisation selon la revendication 9, dans laquelle ledit agent stabilisant microtubulaire est choisi parmi un paclitaxel, des dérivés de paclitaxel, de verapamile, de colchicine et de dexaméthasone.

11. Utilisation selon l'une quelconque des revendications 1 à 7, dans laquelle ladite composition est utilisée conjointement à un traitement par des rayons.

12. Dispositif de traitement de la resténose comprenant un extenseur portant une quantité efficace au plan thérapeutique d'un inhibiteur de topoisomérase.

13. Dispositif selon la revendication 12, dans lequel ledit extenseur est un extenseur polymère chargé d'un inhibiteur de topoisomérase choisi dans le groupe constitué par la camptothécine, l'irinotécane et le topotécane.

14. Dispositif selon la revendication 12, dans lequel l'extenseur est un extenseur métallique et l'inhibiteur de topoisomérase est incorporé dans une gaine de polymère portée par l'extenseur métallique.

15. Dispositif selon la revendication 12, dans lequel l'extenseur est revêtu d'un polymère synthétique ou d'un biopolymère portant l'inhibiteur de topoisomérase.

16. Dispositif selon la revendication 12, dans lequel l'extenseur est un extenseur métallique et l'inhibiteur de topoisomérase est incorporé dans des entailles formées sur l'extenseur.

17. Dispositif selon la revendication 12, qui comprend, en outre, un deuxième agent thérapeutique pour le traitement de la resténose.

18. Dispositif selon la revendication 17, dans lequel ledit deuxième agent thérapeutique est un agent stabilisant microtubulaire.

19. Dispositif selon la revendication 18, dans lequel ledit agent stabilisant microtubulaire est choisi dans le groupe constitué par un paclitaxel, des dérivés de paclitaxel, de verapamile, de colchicine et de dexaméthasone.
